# EUROPEAN PATENT SPECIFICATION

(11) **EP 3 568 122 B1**
(45) Date of publication and mention of the grant of the patent: **04.10.2023**
(21) Application number: 17707659.3
(22) Date of filing: 10.01.2017
(51) Int. Cl.: A61K 9/10, A61K 9/51, A61K 9/14, A61K 33/06, A61K 33/14

(54) **A METHOD OF MANUFACTURING A SUSPENSION OF NANOPARTICLES OF POTASSIUM SALT OR MAGNESIUM SALT**
VERFAHREN ZUR HERSTELLUNG EINER SUSPENSION VON NANOPARTIKELN AUS KALIUMSALZ ODER MAGNESIUMSALZ
PROCÉDÉ DE FABRICATION D'UNE SUSPENSION DE NANOPARTICULES DE SEL DE POTASSIUM OU DE SEL DE MAGNÉSIUM

(43) Date of publication of application: 20.11.2019
(73) Proprietor: Dukebox SP. Z O.O., 01-059 Warszawa (PL)
(72) Inventor: BANACH, Marcin, 32-100 Proszowice (PL); PULIT-PROCIAK, Jolanta, 31-866 Krakow (PL)
(74) Representative: Krekora, Magdalena
(86) International application number: PCT/IB2017/050110
(87) International publication number: WO 2018/130876

(56) References cited:
- EP-A1- 0 222 856
- WO-A1-2014/123935
- WO-A1-2014/203210
- CN-C- 100 506 696
- US-A- 3 539 465
- US-A- 4 822 619
- Manteghian: "JournalofChemicalEngineeringResearchStudi es", , 1 January 2014 (2014-01-01), page 3, XP055385554, Retrieved from the Internet: URL:https://www.researchgate.net/profile/A mir_Faravar2/publication/280079880_Inducti on_time_of_induced_crystallization_of_pota ssium_chloride_nanoparticles/links/55a6bee 208ae92aac77f489e/Induction-time-of-induce d-crystallization-of-potassium-chloride-na noparticles.pdf
- GLAUCIA A. ROCHA-SELMI ET AL: "Double emulsion stage prior to complex coacervation process for microencapsulation of sweetener sucralose", JOURNAL OF FOOD ENGINEERING, vol. 119, no. 1, 1 November 2013 (2013-11-01), pages 28-32, XP055385803, GB ISSN: 0260-8774, DOI: 10.1016/j.jfoodeng.2013.05.002

## Description

### TECHNICAL FIELD

A method of manufacturing a suspension of nanoparticles of potassium salt or magnesium salt.

### BACKGROUND ART

Potassium is a very significant body mineral, important to both cellular and electrical function. The transfer of potassium ions through nerve cell membranes is necessary for normal nerve transmission. Potassium levels influence multiple physiological processes, including resting cellular-membrane potential and the propagation of action potentials in neuronal, muscular, and cardiac tissue; hormone secretion and action; vascular tone; systemic blood pressure control; gastrointestinal motility; acid-base homeostasis; glucose and insulin metabolism; mineralocorticoid action; renal concentrating ability; fluid and electrolyte balance. Potassium salts commonly used in pharmacology are potassium chloride, potassium citrate, potassium permanganate, and potassium gluconate.

Magnesium plays key role in human organism. It is a cofactor in more than 300 enzyme systems that regulate diverse biochemical reactions in the body, including protein synthesis, muscle and nerve function, blood glucose control, and blood pressure regulation. Magnesium is required for energy production, oxidative phosphorylation, and glycolysis. It contributes to the structural development of bone and is required for the synthesis of DNA, RNA, and the antioxidant glutathione. Magnesium also plays a role in the active transport of calcium and potassium ions across cell membranes, a process that is important to nerve impulse conduction, muscle contraction, and normal heart rhythm. Magnesium salts commonly used in pharmacology are magnesium chloride, magnesium citrate, magnesium gluconate, magnesium ascorbate, and magnesium malate.

Patent application EP0766927A1 discloses a salt mixture poor in sodium, comprising also potassium and magnesium and a mixture of vegetables and spices and if desired nutritionally acceptable additives for the substitution of dietary salt, which comprises 20 to 40 % by weight of sodium chloride, 20 to 40 % by weight of potassium chloride, 10 to 20 % by weight of potassium citrate, 0.6 to 1 % by weight of nutritionally acceptable organic magnesium salt, 10 to 15 % by weight of a mixture of vegetables and spices, 5 to 8 % by weight of sodium glutamate and, if desired, 0.03 to 0.20 % by weight of nutritionally acceptable antioxidant, and, if desired, 1 to 3 % by weight of nutritionally acceptable lubricating agent, preferably starch as well as, if desired, other nutritionally acceptable additives, preferably a sugar and/or a sweetening agent. The mixtures according to the invention are biologically active with a curative effect. If consumed by healthy people they promote the healthy nutrition and serve among others for the prevention of cardiovascular disorders. On the other hand for sick people the use of the salt mixtures of the invention may serve as a part of the therapy.

Patent application US4582705A1 discloses a formulation in dosage unit form having the following composition: 5-50 grams of at least one magnesium salt selected from the group consisting of magnesium chloride, magnesium sulfate, magnesium citrate and other suitable magnesium salts; 500 mg to 2 g of at least one potassium salt selected from the group consisting of potassium citrate, potassium sulfate, potassium chloride, potassium bromide, potassium bitartrate and other suitable potassium salts; and 50 mg to 5 grams of at least one salt selected from the group consisting of the citrates, ascorbates, chlorides, bromides, sulfates, carbonates, gluconates, lactates and bitartrates of calcium, sodium, zinc, copper and lithium.

Patent application WO2014108758A1 discloses nanoparticles dispersion is provided for diagnosing skin health conditions comprising at least three components selected from glucose, chloroauric acid, potassium hydroxide, sodium borohydride, and water, wherein said nanoparticle dispersion has a particle size of 1 to 100 nm.

Patent application US20050196434A1 discloses a transdermal pharmaceutical composition which includes a therapeutically effective amount of a pharmaceutically acceptable salt of magnesium and a pharmaceutically acceptable carrier. The pharmaceutically acceptable carrier used in the transdermal pharmaceutical composition of the invention preferably includes a pluronic lecithin organogel.

Patent application RU2306141A1 discloses medicine which contains (g): magnesium nanoparticles 0.001-0.5; polyethylene glycol (PEG)-1500 20-40; glycerol 10; nipagin 0.1, water 5-15; PEG-400 up to 100. Such medicine represents suspension of magnesium nanoparticles in solution of PEG, glycerol and water.

Patent application DE102006002877A1 discloses a method of preparing very small organic nanoparticle, which comprises mixing concentrated and/or saturated solution of the organic compound for the production of organic particle in a water-miscible organic solvent, e.g. ethanol, for precipitating with an aqueous phase, comprising deionized water and/or inorganic salts, e.g. potassium chloride, optionally under the application of ultrasound.

Patent application WO2014123935A1 discloses a method obtaining biodegradable and clinically-compatible nanoparticles as drug delivery carriers based on double-emulsion process. Solution of magnesium chloride is mixed with plasmid DNA coded for the green fluorescence protein (GFP) before being added to cyclohexane/Igepal under vigorous stirring. At the same time, the second emulsion is prepared from sodium phosphate. After forming of magnesium phosphate cores, loaded with DNA, they are dried and subjected to further specified processes.

Patent application WO2014203210A1 discloses a method of obtaining of nanoparticulate formulation comprising a transient receptor potential ankyrin-1 receptor (trpa1) antagonist. The process comprises the step of mixing the active substance or its pharmaceutical salt with surface stabilizer and reducing the particle size of obtained mixture.

Patent specification CN100506696C discloses a method of obtaining of magnesium carbonate three-dimensional nanostructures. The method involves use of urea, and comprises many steps such as stirring for 3 to 8 hours, aging for 1 to 3 hours, filtering and drying the precipitate for 3 hours.

Publication Manteghian M* and Faravar A Induction time of induced crystallization of potassium chloride nanoparticles Journal of Chemical Engineering Research Studies", 2014, 1 (1),103, 1-4 discloses a method of obtaining of potassium chloride nanoparticles in the presence of polyvinylpyrrolidone (PVP) as dispersing agent. This method refers to preparing aqueous solution of PVP (dispersing agent), adding potassium chloride in the solid form, mixing the resulting solution and adding acetone which is used as antisolvent.

Patent application US3539465A discloses encapsulation of hydrophilic liquid-in-oil emulsions. Potassium chloride serves here as coacervating agent. The final product are used as a source of nitrogen with its prolonged releasing for agricultural application. The form of product is a dried powder.

### DISCLOSURE OF THE INVENTION

The method in accordance with the present invention solves the problem of preparation of a stable suspension of nanoparticles of potassium salt or magnesium salt and thus improving their bioavailability and efficacy.

A method of manufacturing a suspension of nanoparticles of potassium salt or magnesium salt of the present invention consists in that, a water solution of potassium salt or magnesium salt, a water solution of lecithin, and a solution of dispersing agents in oil are prepared, then under ultrasound conditions a water solution of lecithin is added to a solution of at least one dispersing agent in oil, and then a solution of potassium salt or magnesium salt and ethanol are added concurrently to the obtained mixture.

Preferably the oil is castor oil.

Preferably the potassium salt or the magnesium salt is a potassium chloride or a magnesium chloride.

Preferably the concentration of ions of the magnesium salt in the suspension is from 50 to 300 mg/cm³ or the concentration of ions of the potassium salt in the suspension is from 50 to 100 mg/cm³.

Preferably the concentration of ions of the magnesium salt in the water solution is from 95 to 600 mg/cm³ or the concentration of ions of the potassium salt in the suspension is from 95 to 200 mg/cm³.

Preferably the concentration of lecithin in the water solution is from 15 to 30%.

Preferably the volume ratio of lecithin solution to the solution of ions of magnesium or potassium is from 0.05 to 0.1.

Preferably characterized in that, the dispersing agent is xanthan gum or/and starch.

Preferably the concentration of xanthan gum in oil is from 1.5 to 2% or/and the concentration of starch in oil is from 4.5 to 6%.

Preferably the ratio of oil to water in the suspension is from 0.35 to 0.55.

Preferably the concentration of ethanol in the suspension is from 10 to 25%.

Preferably the suspension is homogenised through the use of ultrasonic frequencies.

Preferably the strength of ultrasound is from 500 to 600 W.

Preferably the homogenization process is carried out from 1 to 15 minutes.

Preferably the ultrasonication is carried out in flow system or in batch system.

The present disclosure refers also to a suspension of nanoparticles of potassium salt or magnesium salt obtained by method according to the invention.

### BEST MODE OF CARRYING OUT THE INVENTION

The following examples illustrate the invention.

### Example 1

A solution of potassium chloride was prepared by dissolving 30 g of KCl in 80 g of water. A solution of lecithin was prepared by dissolving 1 g of lecithin in 5 g of water in temperature 80°C. Then a solution of dispersing agent was prepared by adding 0.6 g of xanthan gum to 33.5 g castor oil. Under ultrasound conditions at first a solution of lecithin was added portion-wise to the oil-based solution, and then at the same time the solution of potassium chloride and 31,6 g of ethanol were added. Afterwards homogenization was continued for 10 minutes. A homogeneous water suspension of potassium chloride was obtained comprising potassium chloride nanoparticles, having concentration of potassium ions in the amount of 100 mg/cm³.

The Malvern Zetasizer apparatus using method of dynamic light scattering (DLS) was utilized to measure the average size of nanoparticles. The average size of nanoparticles was 153 nm. Potential zeta was equal to -45 mV. The absolute value of the electrokinetic (zeta) potential confirms high stability of the suspension.

### Example 2

A solution of potassium chloride was prepared by dissolving 15 g of KCl in 85 g of water. A solution of lecithin was prepared by dissolving 1 g of lecithin in 5 g of water in temperature 80°C. Then a solution of dispersing agent was prepared by adding 2 g of starch to 33.5 g castor oil. Under ultrasound conditions at first a solution of lecithin was added portion-wise to the oil-based solution, and then at the same time the solution of potassium chloride and 31,6 g of ethanol were added. Afterwards homogenization was continued for 10 minutes. A homogeneous water suspension of potassium chloride was obtained comprising potassium chloride nanoparticles, having concentration of potassium ions in the amount of 50 mg/cm³.

The Malvern Zetasizer apparatus using method of dynamic light scattering (DLS) was utilized to measure the average size of nanoparticles. The average size of nanoparticles was 231 nm. Potential zeta was equal to -70 mV. The absolute value of the electrokinetic (zeta) potential confirms high stability of the suspension.

### Example 3

A solution of potassium chloride was prepared by dissolving 133,8 g of MgCl₂·6H₂O in 80 g of water. A solution of lecithin was prepared by dissolving 1 g of lecithin in 5 g of water in temperature 80°C. Then a solution of dispersing agent was prepared by adding 0.6 g of xanthan gum and 2 g of starch to 33.5 g castor oil. Under ultrasound conditions at first a solution of lecithin was added portion-wise to the oil-based solution, and then at the same time the solution of magnesium chloride and 30 g of ethanol were added. Afterwards homogenization was continued for 15 minutes. A homogeneous water suspension of magnesium chloride was obtained comprising magnesium chloride nanoparticles, having concentration of Mg²⁺ in the amount of 100 mg/cm³.

The Malvern Zetasizer apparatus using method of dynamic light scattering (DLS) was utilized to measure the average size of nanoparticles. The average size of nanoparticles was 139 nm. Potential zeta was equal to -29 mV. The absolute value of the electrokinetic (zeta) potential confirms high stability of the suspension.

## Claims

1. A method of manufacturing a suspension of nanoparticles of potassium salt or magnesium salt, **characterised in that**, a water solution of potassium salt or magnesium salt, a water solution of lecithin, and a solution of dispersing agents in oil are prepared, then under ultrasound conditions a water solution of lecithin is added to a solution of at least one dispersing agent in oil, and then a solution of potassium salt or magnesium salt and ethanol are added concurrently to the obtained mixture.

2. A method according to claim 1, **characterized in that**, the oil is castor oil.

3. A method according to claim 1 or 2, **characterized in that**, the potassium salt or the magnesium salt is a potassium chloride or a magnesium chloride.

4. A method according to claim 3, **characterized in that**, the concentration of ions of the magnesium salt in the suspension is from 50 to 300 mg/cm³ or the concentration of ions of the potassium salt in the suspension is from 50 to 100 mg/cm³.

5. A method according to claim 4, **characterized in that**, the concentration of ions of the magnesium salt in the water solution is from 95 to 600 mg/cm³ or the concentration of ions of the potassium salt in the suspension is from 95 to 200 mg/cm³.

6. A method according to any of the preceding claims, **characterized in that**, the concentration of lecithin in the water solution is from 15 to 30%.

7. A method according to claim 6, **characterized in that** the volume ratio of lecithin solution to the solution of ions of magnesium or potassium is from 0.05 to 0.1.

8. A method according to any of the preceding claims, **characterized in that**, the dispersing agent is xanthan gum or/and starch.

9. A method according to claim 8, **characterized in that**, the concentration of xanthan gum in oil is from 1.5 to 2% or/and the concentration of starch in oil is from 4.5 to 6%.

10. A method according to any of the preceding claims, **characterized in that** the ration of oil to water in the suspension is from 0.35 to 0.55.

11. A method according to any of the preceding claims, **characterized in that**, the concentration of ethanol in the suspension is from 10 to 25%.

12. A method according to any of the preceding claims, **characterized in that**, the suspension is homogenised through the use of ultrasonic frequencies.

13. A method according to claim 12, **characterized in that**, the strength of ultrasound is from 500 to 600 W.

14. A method according to claim 12 or 13, **characterized in that**, the homogenization process is carried out from 1 to 15 minutes.

15. A method according to claim 12 or 13 or 14, **characterized in that**, the ultrasonication is carried out in flow system or in batch system.

## Patentansprüche

1. Verfahren zur Herstellung einer Suspension von Nanopartikeln aus Kalium- oder Magnesiumsalz, **dadurch gekennzeichnet, dass** eine wässrige Lösung von Kalium- oder Magnesiumsalz, eine wässrige Lösung von Lecithin und eine Lösung von Dispergiermitteln in Öl herstellt werden, dann wird unter Ultraschallbedingungen eine wässrige Lösung von Lecithin zu einer Lösung von mindestens einem Dispergiermittel in Öl hinzugefügt und dann gleichzeitig eine Lösung von Kalium- oder Magnesiumsalz und Ethanol zu der erhaltenen Mischung hinzugefügt.

2. Verfahren nach Anspruch 1, **dadurch gekennzeichnet, dass** das Öl Rizinusöl ist.

3. Verfahren nach Anspruch 1 oder 2, **dadurch gekennzeichnet, dass** das Kaliumsalz oder das Magnesiumsalz ein Kaliumchlorid oder ein Magnesiumchlorid ist.

4. Verfahren nach Anspruch 3, **dadurch gekennzeichnet, dass** die Ionenkonzentration des Magnesiumsalzes in der Suspension zwischen 50 und 300 mg/cm3 oder die Ionenkonzentration des Kaliumsalzes in der Suspension zwischen 50 und 100 mg/cm³ liegt.

5. Verfahren nach Anspruch 4, **dadurch gekennzeichnet, dass** die Ionenkonzentration des Magnesiumsalzes in der Wasserlösung 95 bis 600 mg/cm³ oder die Ionenkonzentration des Kaliumsalzes in der Suspension 95 bis 200 mg/cm³ beträgt.

6. Verfahren nach einem der vorhergehenden Ansprüche, **dadurch gekennzeichnet, dass** die Konzentration an Lecithin in der wässrigen Lösung 15 bis 30 % beträgt.

7. Verfahren nach Anspruch 6, **dadurch gekennzeichnet, dass** das Volumenverhältnis der Lecithinlösung zur Lösung der Magnesium- oder Kaliumionen 0,05 bis 0,1 beträgt.

8. Verfahren nach einem der vorhergehenden Ansprüche, **dadurch gekennzeichnet, dass** das Dispergiermittel Xanthangummi oder/und Stärke ist.

9. Verfahren nach Anspruch 8, **dadurch gekennzeichnet, dass** die Konzentration von Xanthangummi in Öl 1,5 bis 2 % beträgt oder/und die Konzentration von Stärke in Öl 4,5 bis 6 % beträgt.

10. Verfahren nach einem der vorhergehenden Ansprüche, **dadurch gekennzeichnet, dass** das Verhältnis von Öl zu Wasser in der Suspension von 0,35 bis 0,55 beträgt.

11. Verfahren nach einem der vorhergehenden Ansprüche, **dadurch gekennzeichnet, dass** die Konzentration von Ethanol in der Suspension 10 bis 25 % beträgt.

12. Verfahren nach einem der vorhergehenden Ansprüche, **dadurch gekennzeichnet, dass** die Suspension durch die Verwendung von Ultraschallfrequenzen homogenisiert wird.

13. Verfahren nach Anspruch 12, **dadurch gekennzeichnet, dass** die Stärke des Ultraschalls zwischen 500 und 600 W liegt.

14. Verfahren nach Anspruch 12 oder 13, **dadurch gekennzeichnet, dass** der Homogenisierungsprozess von 1 bis 15 Minuten durchgeführt wird.

15. Verfahren nach Anspruch 12 oder 13 oder 14, **dadurch gekennzeichnet, dass** die Ultraschallbehandlung im Fließsystem oder im Chargensystem durchgeführt wird.

## Revendications

1. Procédé de fabrication d'une suspension de nanoparticules de sel de potassium ou de sel de magnésium, **caractérisé en ce que**, sont préparées une solution aqueuse de sel de potassium ou de sel de magnésium, une solution aqueuse de lécithine, et une solution d'agents dispersants dans de l'huile, ensuite sous ultrasons est ajoutée une solution aqueuse de lécithine à une solution d'au moins un agent dispersant dans de huile, et ensuite sont ajoutées simultanément une solution de sel de potassium ou de sel de magnésium et de l'éthanol au mélange obtenu.

2. Procédé selon la revendication 1, **caractérisé en ce que**, l'huile est de l'huile de ricin.

3. Procédé selon l'une des revendications 1 ou 2, caractérisé en que, le sel de potassium ou le sel de magnésium est du chlorure de potassium ou du chlorure de magnésium.

4. Procédé selon la revendication 3, **caractérisé en ce que**, la concentration des ions du sel de magnésium dans la suspension est comprise entre 50 et 300 mg/cm³ ou la concentration des ions de sel de potassium dans la suspension est comprise entre 50 et 100 mg/cm³.

5. Procédé selon la revendication 4, **caractérisé en ce que**, la concentration d'ions du sel de magnésium dans la solution aqueuse est comprise entre 95 et 600 mg/cm³ ou la concentration la concentration d'ions du sel de potassium dans la suspension est comprise entre 95 et 200 mg/cm³.

6. Procédé selon l'une quelconque des revendications précédentes, **caractérisé en ce que**, la concentration de lécithine dans la solution aqueuse est comprise entre 15 et 30%.

7. Procédé selon la revendication 6, **caractérisé en ce que** la proportion en volume de solution de lécithine par rapport à la solution d'ions de magnésium ou de potassium est comprise entre 0.05 et 0.1.

8. Procédé selon l'une quelconque des revendications précédentes, caractérisé en que, l'agent dispersant est de la gomme et/ou de l'amidon de xanthane.

9. Procédé selon la revendication 8, caractérisé en que, la concentration de gomme de xanthane dans l'huile est comprise entre 1.5 et 2% et/ou la concentration d'amidon dans l'huile est comprise entre 4.5 et 6%.

10. Procédé selon l'une quelconque des revendications précédentes, caractérisé en que la proportion d'huile par rapport à l'eau dans la suspension est comprise entre 0.35 et 0.55.

11. Procédé selon l'une quelconque des revendications précédentes, caractérisé en que, la concentration d'éthanol dans la suspension est comprise entre 10 et 25%.

12. Procédé selon l'une quelconque des revendications précédentes, caractérisé en que, la suspension est homogénéisée par l'utilisation de fréquences ultrasoniques.

13. Procédé selon la revendication 12, **caractérisé en ce que**, la puissance des ultrasons est comprise entre 500 et 600 W.

14. Procédé selon l'une des revendications 12 ou 13, **caractérisé en ce que**, le procédé d'homogénéisation est réalisé pendant 1 à 15 minutes.

15. Procédé selon l'une des revendications 12 ou 13 ou 14, **caractérisé en ce que**, l'ultrasonification est réalisée par un système de flux ou par un traitement par lots.
